(19)

Europäisches Patentamt
European Patent Office
Office européen des brevets

(11) **EP 4 425 429 B1**

(12) **EUROPEAN PATENT SPECIFICATION**

(45) Date of publication and mention
of the grant of the patent:
**13.05.2026  Bulletin 2026/20**

(21) Application number: **24160545.0**

(22) Date of filing: **29.02.2024**

(51) International Patent Classification (IPC):
**G06T 7/521** *(2017.01)*       **A61C 9/00** *(2006.01)*
**G06T 7/593** *(2017.01)*

(52) Cooperative Patent Classification (CPC):
**G06T 7/521; A61C 9/006; G06T 7/593;**
G06T 2207/10012; G06T 2207/10024;
G06T 2207/30036

(54) **SYSTEM AND METHOD OF SOLVING THE CORRESPONDENCE PROBLEM IN 3D SCANNING SYSTEMS**

SYSTEM UND VERFAHREN ZUR LÖSUNG DES KORRESPONDENZPROBLEMS IN
3D-SCAN-SYSTEMEN

SYSTÈME ET PROCÉDÉ DE RÉSOLUTION DU PROBLÈME DE CORRESPONDANCE DANS DES
SYSTÈMES DE BALAYAGE 3D

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **02.03.2023   DK PA202370115**

(43) Date of publication of application:
**04.09.2024  Bulletin 2024/36**

(73) Proprietor: **3Shape A/S**
**1060 Copenhagen K (DK)**

(72) Inventors:
• **ÖJELUND, Henrik**
  **1060 Copenhagen K (DK)**
• **MOTTELSON, Isak**
  **1060 Copenhagen K (DK)**

(74) Representative: **Zacco Denmark A/S**
**Arne Jacobsens Allé 15**
**2300 Copenhagen S (DK)**

(56) References cited:
WO-A1-2017/003673       CN-A- 110 623 763
CN-A- 114 831 756       US-A1- 2020 404 243
US-A1- 2022 280 269

• BOYER ALAIN ET AL: "Enhancing structured
  light range imaging by adaptation of color,
  exposure and focus", 2017 IEEE
  INTERNATIONAL CONFERENCE ON
  COMPUTATIONAL INTELLIGENCE AND
  VIRTUAL ENVIRONMENTS FOR MEASUREMENT
  SYSTEMS AND APPLICATIONS (CIVEMSA),
  IEEE, 26 June 2017 (2017-06-26), pages 117 - 122,
  XP033134410, DOI: 10.1109/
  CIVEMSA.2017.7995312
• BR◆UER-BURCHARDT CHRISTIAN ET AL: "3D
  reconstruction with single image pairs and
  structured light projection for short-term ultra-
  high-speed applications", PROCEEDINGS OF
  SPIE, IEEE, US, vol. 9528, 18 May 2015
  (2015-05-18), XP060055719, ISBN:
  978-1-62841-730-2, DOI: 10.1117/12.2184704

## Description

## Technical field

The present disclosure relates to a system and method for generating a three-dimensional (3D) representation of an object. In particular, the present disclosure relates to a system and method for determining a solution to the correspondence problem associated with imaging using a 3D scanning device.

## Background

In scanning systems employing triangulation, a central task is to solve the so-called correspondence problem. The correspondence problem may refer to the task of determining image features in images within a set of images and associating each image feature with a projector ray, wherein said images captures the same three-dimensional (3D) scene or object. The images can be taken from a different point of view, at different times, or with objects in a scene in general motion relative to the camera(s).

The correspondence problem can occur in a stereo situation when two images of the same object are acquired, or it can be generalized to an N-view correspondence problem. In the latter case, the images may come from either N different cameras photographing at the same time or from one camera which is moving relative to the object/scene. The problem is made more difficult when the objects in the scene are in motion relative to the camera(s).

In order to generate a three-dimensional digital representation of a scanned object, such as a person's teeth, the correspondence problem generally need to be solved, at least when using a triangulation-based scanning device to acquire the images of the object. In general, it is desired that the 3D digital representation is generated in real-time, or at least in what is perceived as real-time to the user, e.g. the dentist. Therefore, the 3D representation is typically generated simultaneously with the acquisition of images, such that the user can immediately view the generated 3D representation while scanning a patient. Consequently, it is desired that the correspondence problem is solved in a fast, reliable, and accurate manner since it enables a fast generation of the 3D representation.

Thus, it is of interest to develop improved systems and methods for generating a digital 3D representation of a 3D object. In particular, it is desired to find improved methods for solving the correspondence problem and related issues within 3D scanning systems.

US20200404243A1 discloses a method for generating a 3D image includes driving structured light projector(s) to project a pattern of light on an intraoral 3D surface, and driving camera(s) to capture images, each image including at least a portion of the projected pattern, each one of the camera(s) comprising an array of pixels. A processor compares a series of images captured by each camera and determines which of the portions of the projected pattern can be tracked across the images. The processor constructs a three-dimensional model of the intraoral three-dimensional surface based at least in part on the comparison of the series of images. Other embodiments are also described.

US20220280269A1 discloses an apparatus for intraoral scanning comprises an elongate handheld wand comprising a probe at a distal end, one or more light projectors, and two or more cameras. Each light projector comprises at least one light source configured to generate light and a pattern generating optical element configured to generate a pattern of light when the light is transmitted through the pattern generating optical element. Each camera comprises a camera sensor and one or more lenses and is configured to capture a plurality of images that depict at least a portion of the projected pattern of light on an intraoral surface, wherein each camera is configured to focus at an object focal plane that is located between about 1 mm and about 30 mm from a lens of the one or more lenses that is farthest from the camera sensor.

Boyer, Alain & Payeur, Pierre. (2017). Enhancing structured light range imaging by adaptation of color, exposure and focus. 117-122. 10.1109/CIVEMSA.2017.7995312 presents a series of enhancements to a color-coded structured light range sensor that increases the adaptability to complex and unconstrained scenes. First, the projected pattern is made more visible on colored objects by replacing the unique colored pattern with time-multiplexed pseudo-color channels. Second, an exposure fusion algorithm is used when acquiring images to allow the detection of regions with low and high reflectance characteristics. Finally, the focus planes of the scene are automatically detected and imaged separately, enlarging the sensor's depth of field. Each improvement is detailed and integrated into a custom acquisition procedure. Experimental results demonstrate the improved robustness of the structured light range sensor and validate the proposed design.

Bräuer-Burchardt, Christian & Heist, Stefan & Dietrich, Patrick & Kühmstedt, Peter & Notni, Gunther. (2015). 3D reconstruction with single image pairs and structured light projection for short-term ultra-high-speed applications. 952808. 10.1117/12.2184704 discloses a new approach for a 3D reconstruction algorithm using a single pair of a stereo-camera setup and a structured light projection based on spatial correlation is introduced. In comparison to existing methods using sequences of temporally consecutive images, sufficient 3D-reconstruction quality is achieved, even in the case of ultra-high-speed cameras. This is obtained by iterative application of correspondence finding and filtering operators. The calculation effort of the evaluation, filling, filtering, and outlier removing operators is relative high and may prevent a permanent application of the algorithm to high-resolution long-term recordings. The favored application scenario of the new method is the rough 3D reconstruction and motion tracking of quickly moving objects in short-term processes (few seconds), e.g. in the analysis of crash-test situations. Here, the complete recorded image sequence can be analyzed off-line which allows an afterwards optimization of the parameters. An advantage of the new technique regarding high-speed applications is that fixed single patterns instead of pattern sequences can be used for moving objects and hence no synchronization between projection and cameras is necessary.

WO2017003673A1 discloses three-dimensional (3D) scene is computationally reconstructed using a combination of plural modeling techniques. Point clouds representing an object in the 3D scene are generated by different modeled techniques and each point is encoded with a confidence value which reflects a degree of accuracy in describing the surface of the object in the 3D scene based on strengths and weaknesses of each modeling technique. The point clouds are merged in which a point for each location on the object is selected according to the modeling technique that provides the highest confidence.

Summary

While techniques for solving the correspondence problem when projecting a time-varying light pattern exist, the problem becomes more difficult when using a static light pattern. Additional challenges occur when projecting a pattern with a high density of pattern features. Such patterns are generally desired since a high-density pattern improves the resolution of the generated digital representation. However, utilizing a high-density pattern makes the correspondence problem even more difficult to solve, since there is a greater degree of ambiguity. It is an aim of the present disclosure to provide a more robust and accurate way of solving the correspondence problem associated with 3D imaging of an object, in particular in order to obtain a more accurate 3D representation of the scanned object.

The present invention is defined by the independent claim 1. The dependent claims define advantageous embodiments. Further aspects of the present invention will become apparent from the following description with reference to the attached drawings. Any "aspect", "example", and "embodiment" of the description not falling within the scope of the claims does not form part of the invention and is provided for illustrative purposes only.

**The** present disclosure addresses the above-mentioned challenges by providing a scanning system for generating a three-dimensional (3D) representation of an object, the scanning system comprising:

- an intraoral scanning device comprising:

    - one or more projector units configured to project a pattern on a surface of the object;
    - two or more camera units configured to acquire a set of images comprising at least one image from each camera unit, wherein each image is composed of an array of pixels, wherein each pixel comprises a pixel color defined by one or more color channels;

    wherein the scanning system further comprises one or more processors configured for:

    - determining points in three-dimensional (3D) space that form a solution to a correspondence problem associated with the set of images, wherein the points are determined by comparing pixel colors with computed colors associated with camera rays corresponding to each pixel; and
    - generating the three-dimensional (3D) representation based on the determined 3D points.

**The** present disclosure further relates to a computer-implemented method for generating a three-dimensional (3D) representation of a surface of an object, the method comprising the steps of:

- obtaining or acquiring a set of images comprising two or more images, wherein each image comprises a plurality of image features, wherein each image is composed of an array of pixels, wherein each pixel comprises a pixel color defined by one or more color channels;
- determining points in three-dimensional (3D) space that form a solution to a correspondence problem associated with the set of images, wherein the points are determined by comparing pixel colors with computed colors associated with camera rays corresponding to each pixel; and
- generating the three-dimensional (3D) representation based on the determined 3D points.

The present disclosure further relates to a computer-implemented method for determining a solution to a correspondence problem associated with a triangulation-based 3D scanning device, the method comprising the steps of:

- obtaining or acquiring a set of images comprising two or more images, wherein the images are acquired by separate camera units at the same moment in time, wherein each image comprises a plurality of image features, wherein each image is composed of an array of pixels, each pixel comprising one or more color channels;
- determining one or more image features within the set of images, wherein each image feature is associated with a true color corresponding to a given pixel;
- determining potential points in 3D that form one or more candidate solutions to the correspondence problem, wherein each potential point is associated with one or more image features from different camera units;
- assigning one or more parameters to each potential point, wherein said parameters include a color and a likelihood that the potential point is part of the true solution;
- generating a computed color for each pixel based on the assigned colors and likelihoods to the potential points; and
- determining, among the potential points, points in 3D that form a true solution to the correspondence problem by solving an optimization problem, wherein the color and likelihood of the 3D points are determined by minimizing a cost function based on the difference between the computed color and the true color.

The present disclosure further relates to a data processing system, such as the scanning system disclosed herein, comprising one or more processors configured to perform the steps of the method disclosed herein.

The present disclosure further relates to a computer program product comprising instructions which, when the program is executed by a computer, cause the computer to carry out the steps of the method disclosed herein. The present disclosure further relates to a computer-readable data carrier having stored thereon said computer program product.

The present disclosure further relates to a computer-readable storage medium comprising instructions which, when executed by a computer, cause the computer to carry out the steps of the method disclosed herein.

The disclosed system and method provide a more efficient and reliable framework for solving the correspondence problem associated with 3D scanning systems, and consequently a more accurate three-dimensional (3D) representation of the scanned object can be generated. In particular, the disclosed system and method provides an improvement over existing methods, e.g. due to the ability to resolve the ambiguity encountered when solving the correspondence problem, whereby a more accurate 3D representation is obtained.

## Brief description of the drawings

Fig. 1 shows a scanning system according to the present disclosure.
Fig. 2 shows an embodiment of the computer-implemented method disclosed herein.
Fig. 3 shows another embodiment of the computer-implemented method disclosed herein.
Fig. 4 shows yet another embodiment of the computer-implemented method disclosed herein.
Fig. 5 shows a computer system in which embodiments of the present disclosure, or portions thereof, may be implemented as computer-readable code.
Fig. 6 shows a visualization of candidate solutions to the correspondence problem associated with a set of images.

## Detailed description

*Three-dimensional object*

The three-dimensional (3D) object may be a dental object. Examples of dental objects include any one or more of: tooth/teeth, gingiva, implant(s), dental restoration(s), dental prostheses, edentulous ridge(s), and/or combinations thereof. Alternatively, the dental object may be a gypsum model or a plastic model representing a subject's teeth. As an example, the three-dimensional (3D) object may comprise teeth and/or gingiva of a subject. The dental object may only be a part of the subject's teeth and/or oral cavity, since the entire set of teeth of the subject is not necessarily scanned during a scanning session. A scanning session may be understood herein as a period of time during which data (such as images) of the 3D object is obtained.

*Scanning device*

The scanning device disclosed herein may be an intraoral scanning device for acquiring images within an intraoral cavity of a subject. The scanning device may be a handheld scanning device, i.e. a device configured to be held with a human hand. The scanning device may employ any suitable scanning principle such as triangulation-based scanning, stereo vision, structure from motion, confocal scanning, or other scanning principles.

In preferred embodiments, the scanning device employs a triangulation-based scanning principle. As an example, a projector unit and one or more camera units may be utilized to determine points in 3D space based on triangulation. As another example, the scanning device comprises a projector unit and two or more camera units, wherein the camera units are configured to image the scanned object from separate views, i.e. from different directions. In particular, the camera units may be configured to acquire a set of images, wherein a correspondence problem is solved within said set of images based on triangulation. The images within a set of images are preferably acquired simultaneously, i.e. at the same moment in time. The correspondence problem generally refers to the problem of ascertaining which parts of one image correspond to which parts of another image. Specifically, the projector unit may be configured to project a plurality of projector rays, which are projected onto a surface of the dental object. Solving the correspondence problem may include the steps of determining image features in the images within a set of images, and further associate said image features with a specific projector ray. Subsequently, the depth of each projector ray may be computed, whereby a 3D representation of the scanned object may be generated. In some embodiments, the correspondence problem is solved jointly for groups of projector rays, as opposed to e.g. solving the correspondence problem projector ray by projector ray.

The scanning device may comprise one or more scan units, wherein each scan unit comprises a projector unit and one or more camera units. As an example, the scanning device may comprise one scan unit comprising one projector unit and at least two camera units. As another example, the scanning device may comprise one scan unit comprising one projector unit and four camera units. In yet another example, the scanning device may comprise at least two scan units, wherein each scan unit comprises a projector unit and two or more camera units. In yet another example, the scanning device may comprise at least two scan units, wherein each scan unit comprises a projector unit and four camera units.

The scanning device may further comprise a reflecting element arranged in combination with a given scan unit. The reflecting element is preferably configured to reflect light from the projector unit of the scan unit and/or from the surface of the dental object and onto the image sensor(s) of each camera unit of the scan unit associated with the reflecting element. In preferred embodiments, the scanning device comprises or constitutes an elongated probe, which defines a longitudinal axis of the scanning device.

*Projector unit*

A projector unit may be understood herein as a device configured for projecting light onto a surface, such as the surface of a three-dimensional object. In preferred embodiments, the projector unit is configured to project a pattern of light onto the surface of a dental object. Preferably, the projector unit is configured to project a pattern of light such that the pattern of light is in focus at a predefined focus distance measured along a projector optical axis. In some embodiments, the projector unit is configured to project the pattern of light such that the pattern of light is defocused at the opening of the probe of the scanning device and/or at a surface of an optical window in said probe.

The projector unit may comprise Digital Light Processing (DLP) projectors using a micro mirror array for generating a time varying pattern, or a diffractive optical element (DOF), or front-lit reflective mask projectors, or micro-LED projectors, or Liquid crystal on silicon (LCoS) projectors or back-lit mask projectors, wherein a light source is placed behind a mask having a spatial pattern, whereby the light projected on the surface of the dental object is patterned. The pattern may be dynamic, i.e. such that the pattern changes over time, or the pattern may be static in time, i.e. such that the pattern remains the same over time. The projector unit may comprise one or more collimation lenses for collimating the light from the light source. The collimation lens(es) may be placed between the light source and the mask. The projector unit may further comprise one or more focus lenses, or lens elements, configured for focusing the light at a predefined focus distance.

In some embodiments, the projector unit of the scanning device comprises at least one light source and a pattern generating element for defining a pattern of light. The pattern generating element is preferably configured for generating a light pattern to be projected on a surface of a dental object. As an example, the pattern generating element may be a mask having a spatial pattern. Hence, the projector unit may comprise a mask configured to define a pattern of light. The mask may be placed between the light source of the projector unit and the one or more focus lenses, such that light transmitted through the mask is patterned into a light pattern. As an example, the mask may define a polygonal pattern comprising a plurality of polygons, such as a checkerboard pattern. The projector unit may further comprise one or more lenses such as collimation lenses or projection lenses. In other embodiments, the pattern generating element is based on diffraction and/or refraction to generate the light pattern, such as a pattern comprising an array of discrete unconnected dots.

The projector unit may be configured to generate a predefined static pattern, which may be projected onto a surface of an object. An advantage of using a static pattern is that it enables the ability to capture all image data simultaneously, thus preventing warping due to movement. Another advantage is that a static pattern makes it possible to acquire only one image per camera unit in the set of images, thereby reducing power consumption e.g. of the light source.

Alternatively, the projector unit may be configured to generate a dynamic pattern, which changes in time. The projector unit may be associated with its own projector plane, which is determined by the projector optics. As an example, if the projector unit is a back-lit mask projector, the projector plane may be understood as the plane wherein the mask is contained. The projector plane comprises a plurality of pattern features of the projected pattern. Preferably, the camera units and projector unit are arranged such that the image sensors and the projector plane, e.g. defined by the mask, are in the same plane.

The projector unit may define a projector optical axis. An optical axis may be understood as a line along which there is some degree of rotational symmetry in an optical system such as a camera lens or a projector unit. In some embodiments, the projector optical axis of the projector unit is substantially parallel with the longitudinal axis of the scanning device. In other embodiments, the projector optical axis of the scan unit defines an angle, such as at least 45° or at least 75°, with the longitudinal axis of the scanning device. In other embodiments, the projector optical axis of the projector unit is substantially orthogonal to the longitudinal axis of the scanning device.

*Light source*

The projector unit may comprise one or more light sources. The projector unit may be configured to project a pattern of light defined by a plurality of projector rays when the light source(s) are on/active. The light source(s) may be configured to generate light of a single wavelength or a combination of wavelengths (mono- or polychromatic). The combination of wavelengths may be produced by a light source configured to produce light comprising different wavelengths (such as white light).

In some embodiments, each projector unit comprises a light source for generating white light. Alternatively, the projector unit may comprise multiple light sources such as LEDs individually producing light of different wavelengths (such as red, green, and blue) that may be combined to form light comprising different wavelengths. Thus, the light produced by the light source(s) may be defined by a wavelength defining a specific color, or a range of different wavelengths defining a combination of colors such as white light. In some embodiments, the light source is a diode, such as a white light diode, or a laser diode.

In some embodiments, the scanning device comprises a light source configured for exciting fluorescent material to obtain fluorescence data from the dental object such as from teeth. Such a light source may be configured to produce a narrow range of wavelengths. In other embodiments, the scanning device comprises an infrared light source, which is configured to generate wavelengths in the infrared range, such as between 700 nm and 1.5 $\mu$m. In some embodiments, the scanning device comprises one or more light sources selected from the group of: Infrared (IR) light source, near-infrared (NIR) light source, blue light source, violet light source, ultraviolet (UV) light source, and/or combinations thereof. In some embodiments, the scanning device comprises a first light source forming part of the projector unit, and one or more second light sources, e.g. IR-LED(s) and/or UV-LED(s), located in a distal part of the scanning device, such as in the tip of the scanning device.

*Pattern of light*

The projector unit may be configured to project a pattern of light defined by a plurality of projector rays when a light source of the projector unit is turned on. The terms 'pattern of light', 'spatial pattern', and 'pattern' are used herein interchangeably. The pattern may be generated using a pattern generating element, e.g. located in the projector unit. The pattern generating element may be a mask, such as a transparency or transmission mask, having a spatial pattern. In other embodiments, the pattern generating element is configured to utilize diffraction and/or refraction to generate a light pattern.

The spatial pattern may be a polygonal pattern comprising a plurality of polygons. The polygons may be selected from the group of: triangles, rectangles, squares, pentagons, hexagons, and/or combinations thereof. Other polygons can also be envisioned. In general, the polygons are composed of edges and corners. In some embodiments, the polygons are repeated in the pattern in a predefined manner. As an example, the pattern may comprise a plurality of repeating units, wherein each repeating unit comprises a predefined number of polygons, wherein the repeating units are repeated throughout the pattern. Alternatively, the pattern may comprise a predefined arrangement comprising any of stripes, squares, dots, triangles, rectangles, and/or combinations thereof. In some embodiments, the pattern is non-coded, such that no part of the pattern is unique.

In some embodiments, the generated pattern of light is a polygonal pattern, such as a checkerboard pattern comprising a plurality of checkers. Similar to a common checkerboard, the checkers in the pattern may have alternating dark and bright areas corresponding to areas of low light intensity (dark) and areas of high(er) light intensity (bright). In some embodiments the pattern of light is a checkerboard pattern comprising alternating squares of dark and bright. In some embodiments, the light pattern comprises a distribution of discrete unconnected spots of light.

The pattern preferably comprises a plurality of pattern features. The pattern may be a high-density pattern, which may be understood as a pattern comprising more than 3000 pattern features. However, the presently disclosed system and method is not limited to high-density patterns, since it works equally well for patterns of lower density. In some embodiments, the pattern comprises at least 1000 pattern features, or at least 3000 pattern features, or at least 10000 pattern features. When projecting a pattern comprising pattern features onto a surface of the 3D object, the acquired images of the object will similarly comprise a plurality of image features corresponding to the pattern features. A pattern/image feature may be understood as an individual well-defined location in the pattern/image. Examples of image/pattern features include corners, edges, vertices, points, transitions, dots, stripes, etc. In some embodiments, the image/pattern features comprise the corners of checkers in a checkerboard pattern. In other embodiments, the image/pattern features comprise corners in a polygon pattern such as a triangular pattern.

*Camera unit*

A camera unit may be understood herein as a device for capturing an image of an object. Each camera unit may comprise an image sensor for generating an image based on incoming light e.g. received from an illuminated 3D object. As an example, the image sensor may be an electronic image sensor such as a charge-coupled device (CCD) or an active-pixel sensor (CMOS sensor). In some embodiments, the image sensor is a global shutter sensor configured to expose the entire image area (all pixels) simultaneously and generate an image in a single point in time. The image sensor may have an image frame rate of at least 30 frames per second, such as at least 60 frames per second, or even at least 90 frames per second.

The image sensor(s) may comprise an array of pixels, wherein each pixel is associated with a corresponding camera ray. Similarly, each image, within the set of images, may be composed of an array of pixels, wherein each pixel comprises a pixel color defined by one or more color channels. The array of pixels may be a two-dimensional (2D) array. In some embodiments, the image sensor is a CMOS sensor comprising an analog-to-digital converter (ADC) for each column of pixels, making conversion time significantly faster and allowing each camera unit to benefit from greater speed. Each image sensor may define an image plane, which may be understood as the plane that contains the object's projected image. Each image obtained by the image sensor(s) may comprise a plurality of image features, wherein each image feature originates from a pattern feature of the projected pattern. In some embodiments, one or more of the camera units comprise a light field camera. Preferably, each camera unit defines a camera optical axis. The camera units may further comprise one or more focus lenses for focusing light.

In some embodiments, the image sensor is a monochrome image sensor, wherein each pixel is associated with a single color channel, e.g. is a grayscale color channel, wherein the value of each pixel represents only an amount of light. In other embodiments, the image sensor is a color image sensor or an image sensor comprising a color filter array on the array of pixels. As an example, the color filter array may be a Bayer filter employing an arrangement of four color filters: Red (R), Green (G), Green (G), and Blue (B). The Bayer filter may also be referred to as a RGGB filter. When utilizing the image sensor data, color pixels may be combined to monochrome pixels of 2 x 2 color pixels for 3D depth reconstruction. In this case, the resolution of the 3D depth reconstruction is only half the resolution of the image sensor in each direction. When obtaining texture (color) images the full native resolution is preferably utilized (with color filtered pixels).

In accordance with some embodiments, the projector optical axis and the camera optical axis of at least one camera unit define a camera-projector angle of approximately 5 to 15 degrees, preferably 5 to 10 degrees, even more preferably 8 to 10 degrees. In some embodiments, the camera units are defocused at the opening of the probe of the scanning device and/or at the surface of an optical window in said probe. In some embodiments of the scanning device, the camera units and projector unit of a given scan unit are focused at the same distance. In some embodiments, each camera unit has a field of view of 50-115 degrees, such as 65-100 degrees, or 80-90 degrees.

*Scan unit*

A scan unit may be understood herein as a unit comprising at least one projector unit and one or more camera units. In some embodiments, each scan unit comprises at least two camera units having at least partly overlapping fields of view along different camera optical axes. Preferably, each scan unit comprises at least four camera units having at least partly overlapping fields of view along different camera optical axes.

*Reflecting element*

A reflecting element may be understood herein as an element configured to change the direction of light rays incident on the surface of said reflecting element or being transmitted through said reflecting element, e.g. in case of a prism. In particular, the reflecting element is preferably configured to change the direction of a center beam of the projected light from a projector unit from a direction substantially parallel to the longitudinal axis of the scanning device to a direction substantially orthogonal to said longitudinal axis. In some embodiments, a surface normal of each reflecting element defines an angle with respect to the projector optical axis of approximately 40-50 degrees, preferably approximately 45 degrees.

As an example, the reflecting element may be selected from the group of: mirrors, prisms, and/or combinations thereof. In some embodiments, the reflecting element is configured to reflect light from the projector unit of the scan unit and/or reflect light from the surface of the object being scanned and onto the image sensors of the scan unit. In some embodiments, the scanning device comprises a mirror as the reflecting element. In other embodiments, the scanning device comprises a prism as the reflecting element. Some embodiments feature a combination of mirror(s) and prism(s). In case of a prism as the reflecting element, the prism is preferably configured to change the direction of a center beam of the projected pattern of light from substantially parallel to the longitudinal axis of the scanning device to a direction having an angle of at least 45 degrees with respect to said longitudinal axis. Even more preferably, the prism is configured to change the direction of a center beam of the projected pattern of light from substantially parallel to the longitudinal axis of the scanning device to a direction having an angle of approximately 90 degrees with respect to said longitudinal axis.

In some embodiments, the scanning device comprises a scan unit, wherein the scanning device further comprises a reflecting element positioned on the projector optical axis of the projector unit of said scan unit. In other embodiments, the scanning device comprises at least two scan units, wherein the scanning device further comprises a reflecting element arranged in combination with each scan unit. The reflecting element is then configured to reflect light projected from the projector unit of said scan unit. The reflecting element is preferably further arranged to reflect light from the object being scanned and onto the image sensor(s) of each camera unit of the scan unit. In some embodiments, the reflecting element of each scan unit is positioned on the projector optical axis. The projector optical axis may in some embodiments coincide with the longitudinal axis of the scanning device.

*Processor*

In accordance with some embodiments, the scanning device comprises one or more processors selected from the group of: central processing units (CPU), accelerators (offload engines), general-purpose microprocessors, graphics processing units (GPU), neural processing units (NPU), application-specific integrated circuits (ASIC), field-programmable gate arrays (FPGA), dedicated logic circuitry, dedicated artificial intelligence processor units, or combinations thereof.

The scanning device may further comprise computer memory for storing instructions, which when executed, causes the processor(s) to carry out the step of determining image features in the set of images. The computer memory may further store instructions, which when executed, causes the processor(s) to carry out the step of generating a digital representation of a three-dimensional (3D) object. In general, the processor(s) may be configured to perform any of the disclosed computer-implemented methods herein, either fully or in part, e.g. such that some processor(s) perform some method steps and other processors perform other method steps, when executed.

The processor(s) may both be located on the scanning device. Alternatively, the processor(s), or a sub-set of the processor(s), may be located on a computer system as described herein. As an example, a first processor may be configured to determine image features in the images, and subsequently provide data related to the determined image features to a second processor. The data may comprise image feature coordinates as well as other attributes such as a camera index or other predefined properties of the image features.

The processor(s) may be configured to generate the digital representation of the 3D object, e.g. in the form of a point cloud. The scanning device may be further configured to provide the digital representation to a computer system for rendering the representation. The computer system may further process the digital representation, e.g. by stitching the point clouds received from the scanning device and/or by fitting one or more surfaces to the stitched point clouds. This further processing by the computer system may also be referred to herein as reconstruction. The output of the reconstruction is a digital 3D model of the scanned object. The digital 3D model may be rendered and displayed on a display, e.g. connected to the computer system.

*Module for transmitting data*

The scanning device may comprise a module for transmitting data, such as images or point clouds, to one or more external devices, such as to a computer system. The module may be a wireless module configured to wirelessly transfer data from the scanning device to the computer system. The wireless module may be configured to perform various functions required for the scanning device to wirelessly communicate with a computer network. The wireless module may utilize one or more of the IEEE 802.11 Wi-Fi protocols/ integrated TCP/IP protocol stack that allows the scanning device to access the computer network. It may also use other network The wireless module may include a system-on-chip having different types of inbuilt network connectivity technologies. These may include commonly used wireless protocols such as Bluetooth, ZigBee, Wi-Fi, WiGig (also known as 60 GHz Wi-Fi), etc. The scanning device may further (or alternatively) be configured to transmit data using a wired connection, such as an ethernet cable or a USB cable. In some embodiments, the scanning device comprises a wireless module configured to wirelessly transfer data from the scanning device to the computer system.

*Computer system*

A computer system may be understood as an electronic processing device for carrying out sequences of arithmetic or logical operations. In the present context, a computer system refers to one or more devices comprising at least one processor, such as a central processing unit (CPU), along with some type of computer memory. Examples of computer systems falling within this definition include desktop computers, laptop computers, computer clusters, servers, cloud computers, quantum computers, mobile devices such as smartphones and tablet computers, and/or combinations thereof.

The computer system may comprise hardware such as one or more central processing units (CPU), graphics processing units (GPU), and computer memory such as random-access memory (RAM) or read-only memory (ROM). The computer system may comprise a CPU, which is configured to read and execute instructions stored in the computer memory e.g. in the form of random-access memory. The computer memory is configured to store instructions for execution by the CPU and data used by those instructions. As an example, the memory may store instructions, which when executed by the CPU, cause the computer system to perform, wholly or partly, any of the computer-implemented methods disclosed herein. The computer system may further comprise a graphics processing unit (GPU). The GPU may be configured to perform a variety of tasks such as video decoding and encoding, rendering of the digital representation, and other image processing tasks.

The computer system may further comprise non-volatile storage in the form of a hard disc drive. The computer system preferably further comprises an I/O interface configured to connect peripheral devices used in connection with the computer system. More particularly, a display may be connected and configured to display output from the computer system. The display may for example display a 2D rendering of the generated digital 3D representation. Input devices may also be connected to the I/O interface. Examples of such input devices include a keyboard and a mouse, which allow user interaction with the computer system. A network interface may further be part of the computer system in order to allow it to be connected to an appropriate computer network so as to receive and transmit data (e.g. scan data, sub-scans, and/or images) from and to other computing devices. The CPU, volatile memory, hard disc drive, I/O interface, and network interface, may be connected together by a bus.

The computer system is preferably configured for receiving data from the scanning device, either directly from the scanning device or via a computer network such as a wireless network. The data may comprise images, processed images, sub-scans, point clouds, sets of data points, or other types of data. The data may be transmitted/received using a wireless connection, a wired connection, and/or combinations thereof. In some embodiments, the computer system is configured for generating a digital representation of a three-dimensional (3D) object as described herein. In some embodiments, the computer system is configured for receiving data, such as sub-scans or point clouds, from the scanning device and then subsequently perform the steps of reconstruction and rendering a digital representation of a three-dimensional (3D) object. Rendering may be understood as the process of generating one or more images from three-dimensional data. The computer system may comprise computer memory for storing a computer program, said computer program comprising computer-executable instructions, which when executed, causes the computer system to carry out the method of generating a digital representation of a three-dimensional (3D) object.

*Acquisition* of *images*

The method disclosed herein may comprise the step of acquiring or obtaining a set of images. A set of images may be understood herein as comprising two or more two-dimensional (2D) images. The 2D images may be digital images, such as digital color images. The set of images may be acquired by the scanning system disclosed herein, such as by an intraoral scanning device. The intraoral scanning device may comprise multiple camera units, such as two or more camera units, or four or more camera units. Each camera unit may comprise an image sensor having an array of pixels. Each pixel may be associated with a corresponding camera ray in three-dimensional (3D) space originating from said pixel. Generally, a point located along a given camera ray corresponding to a given pixel will be imaged on the image sensor on said pixel.

Each 2D image may be composed of an array of pixels, such as a two-dimensional array of pixels, corresponding to the array of pixels on the image sensor(s). Each pixel may be associated with a camera ray as described above. Each pixel may comprise a pixel color defined by one or more color channels. An example of a single color channel is a grayscale color channel, wherein the value of each pixel represents only an amount of light, thereby carrying intensity information. Thus, the images within the set of images may be grayscale images. Another example of a single color channel is red, green, or blue. Thus, the presently disclosed method is not limited to using pixel color information, but may instead utilize pixel intensity information.

Alternatively, each pixel may comprise more than one color channel, such as three color channels, e.g. a red, green, and blue channel (RGB). In that case, the images within the set of images may be RGB images. The RGB images may be 24-bit, such that each channel has 8 bits, for red, green, and blue. In other words, the image may be composed of three images (one for each channel), wherein each image can store discrete pixels with brightness intensities between 0 and 255. The RGB images may alternatively be 48-bit, corresponding to a very high color-depth, wherein each channel has 16 bits per pixel color, that is 16-bit red, green, and blue for each pixel. In some embodiments, each sensor pixel comprises a color filter so that it can only capture light of a certain wavelength or narrow range of wavelengths, e.g. such that a given pixel can only capture either red, green, or blue light. The arrangement of color filters over the sensor pixels may constitute a color filter array, such as a Bayer filter. In some cases, a color is assigned to a given image pixel by averaging the color of neighboring sensor pixels.

In some embodiments, each pixel in the array of pixels on the image sensor comprises a pixel color defined by a single color channel, such as red, green, or blue. In such embodiments, the method may comprise the step of acquiring three consecutive sets of images, wherein a first set of images are obtained with e.g. red pixels, a second set of images are obtained with green pixels, and a third set of images are obtained with blue pixels. In that case, a given image does not comprise three color channels; instead the three color channels may be provided by three different sets of images. Thus, color in the image pixels may be inferred from the three consecutive sets of images obtained by the scanning device.

In some embodiments, the set of images are acquired using an intraoral scanning device, such as a triangulation-based intraoral scanning device employing multiple camera units or image sensors. As an example, the set of images may be acquired using an intraoral scanning device comprising two or more camera units, such as four or more camera units. In some embodiments, the set of images comprises four images, wherein images within the set of images are acquired by four different camera units. In other words, each camera unit or image sensor may contribute with one image to the set of images, such that the number of images within the set of images corresponds to the number of camera units or image sensors used to acquire said images. In some embodiments, the images are acquired simultaneously, i.e. at the same moment in time.

*Determining 3D points*

Each image within the set of images may comprise a number of image features, such as a plurality of image features. In some embodiments, the disclosed method comprises the step of determining one or more image features within the set of images. The image features may be associated with pattern features which are part of the pattern projected on the surface of the object. In general, the images may be associated with a correspondence problem, which is desired to solve in order to determine corresponding image features between the images. Thus, a purpose of the presently disclosed system and method is to solve said correspondence problem, whereby a 3D representation of the scanned object may be generated.

The scanning system may be configured to continuously acquire sets of images with a predetermined framerate during a scanning session, wherein one or more objects, such as the teeth of a patient, is scanned using an intraoral scanning device. Preferably, the computer-implemented method disclosed herein is executed for each set of images acquired, such that the correspondence problem associated with said set of images, is solved. In preferred embodiments, said correspondence problem is solved before the acquisition of a subsequent set of images. As an example, if the image sets are acquired with a framerate of 20 image sets per second, then the scanning system may be configured to solve the correspondence problem in a time of less than 1/20 of a second.

The scanning system may comprise an intraoral scanning device comprising one or more projector units configured to project a pattern on a surface of the object. The projected pattern may comprise a plurality of pattern features. In general, the projected pattern is generated by a plurality of projector rays. Each pattern feature may be associated with a corresponding projector ray in three-dimensional (3D) space. In preferred embodiments, the method comprises the step of determining image features within the set of images. The method may further comprise the step of projecting camera rays in 3D space, each camera ray corresponding to a given image feature.

The method may comprise the step of determining potential points in three-dimensional (3D) space that form one or more candidate solutions to the correspondence problem. The potential points may be determined based on triangulation. Specifically, potential points may be determined based on intersections of camera rays with a given projector ray, each camera ray originating from a given image feature in an image. Thus, camera rays may be projected from all the image features in each image. In general, images within the set of images may comprise a number of corresponding image features, whose correspondence is not yet known. As an example, in the case of four images obtained by four separate camera units, a potential point in 3D space may be identified based on four camera rays intersecting a projector ray. The intersections of camera rays and a given projector ray may not form a perfect intersection; rather the camera rays may intersect a projector ray within some tolerance. Bases on said intersections, one or more potential 3D points may be determined for each projector ray. The potential points may be assigned a depth corresponding to the distance measured along a given projector ray originating from the pattern generating element. For each projector ray, there is only one point in 3D space that forms the true solution to the correspondence problem. However, this solution may not be known at this point. Rather, the method may identify one or more potential points for each projector ray, wherein the true solution may be determined among said potential points.

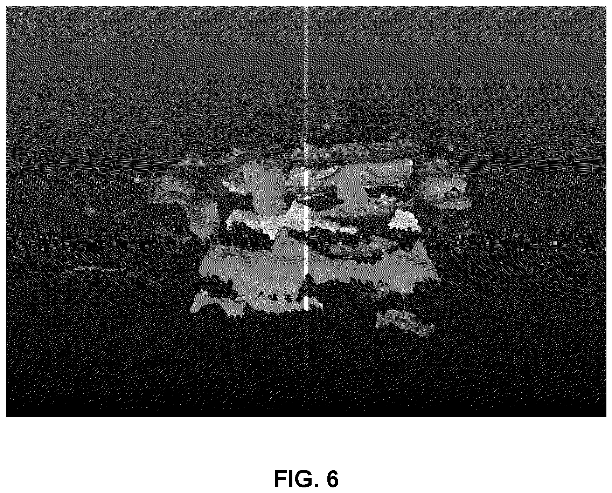

The potential points in 3D may be connected to form one or more meshes or three-dimensional surfaces. Said surfaces form a collection of plausible solutions, or candidate solutions, to the correspondence problem. An example of such candidate solutions are illustrated in figure 6. In general, the complexity of the correspondence problem scales with the density of the projected pattern. Thus, a very dense pattern, corresponding to a pattern having a large number of pattern features, e.g. a high-density pattern, will result in ambiguity when solving the correspondence problem, thereby leading to many plausible solutions to the problem. Thus, a remaining task is to identify the true solution to the problem among the plausible solutions. In other words, the task is to identify 3D points among the potential points, such that a 3D representation of the object can be generated based on said 3D points. The 3D representation may be a point cloud model, a signed distance model, a triangulated point cloud model, a collection of point clouds optionally with additional information such as uncertainty estimates or color(s), a collection of triangulated point clouds, a polygon mesh, a volumetric representation such as a voxel model, a parametrized surface, a surface elements model, or any other suitable three-dimensional representation.

To achieve this, the method may further comprise the step of assigning one or more parameters to each potential point. The parameters may include a color, $c_i$, and a likelihood, $\alpha_i$, that the potential point is part of the solution to the correspondence problem. The likelihood may be expressed as a number between 0 and 1, wherein said number is a measure of how probable/likely it is that the given potential point forms part of the true solution.. It should be noted that the step does not necessarily include assigning specific values of the parameters. The parameters may be understood as mathematical variables. However, the method may comprise the step of providing an initial value for the colors and/or likelihoods.

The method may further comprise the step of generating a computed color for each camera ray corresponding to a pixel, wherein the computed color is based on the assigned colors and likelihoods. Thus, each pixel may similarly be associated with a computed color and a pixel color, wherein the latter is considered the true color of the pixel as given by the one or more color channels in the image. In some embodiments, the computed color of a given camera ray, *r*, is defined by $\widehat{C_r} = \alpha_0 c_0 + \alpha_1 c_1(1 - \alpha_0) + \alpha_2 c_2(1 - \alpha_0)(1 - \alpha_1) + (...)$. This equation may be written in the more generalized and concise form given below:

$$\widehat{C_r} = \sum_{i=1}^{N} \alpha_i c_i T_i$$

where $c_i$ is the color of a given potential point at the *i*'th intersection of a given projector ray, $\alpha_i$ is the likelihood that the potential point is part of the solution, and $T_i$ is a transparency given by $T_i = \prod_{j=1}^{i-1}(1 - \alpha_j)$. $T_i$ is associated with a given potential point and the camera ray associated with that point. $T_i$ is a measure of the likelihood that no prior potential point on the ray is the correct one. Thus, the method may comprise the step of generating a computed color for each pixel, or for each camera ray corresponding to said pixel, based on the assigned colors and likelihoods. Advantageously, the expression for the computed color of a given camera ray is differentiable with respect to $\alpha_i$ and $c_i$. In other words, the computed color is preferably a continuous and differentiable function. Continuous and differentiable may be understood as having their ordinary meaning within the field of mathematics. An advantage hereof is that there exist stronger and more efficient mathematical tools for solving continuous problems than for discrete problems, which consequently makes the disclosed method more efficient in terms of computational time and power. In particular, a continuous and differentiable function enables the use of gradient-based methods. Examples of gradient-based methods are the gradient descent, which is a first-order iterative optimization algorithm for finding a local minimum of a differentiable function, and the conjugate gradient, which is an algorithm for the numerical solution of linear equations, whose matrix is positive-definite.

The method may further comprise the step of determining the numeric value of the color and likelihood of the potential points by minimizing a cost function based on the difference between the computed colors and the pixel colors. As previously mentioned, each pixel in the array of pixels (in the image or on the image sensor) may have a pixel color defined by one or more color channels. Thus, the true color of a given pixel, or camera ray corresponding to that pixel, is known. Instead of utilizing pixel colors, pixel intensities may be utilized, e.g. as defined by a single color channel. By utilizing the expression for the computed color of the camera rays and the true color (e.g. the pixel color or pixel intensity) of the camera rays, the numeric value of the color and likelihood of each potential point may be determined by solving an optimization problem, wherein a cost function is minimized. In some embodiments, the cost function is defined by the equation below:

$$\min_{\alpha, c} \sum_{r} \left\| C_r - \widehat{C_r} \right\|^2$$

where $C_r$ is the pixel color of the pixel corresponding to a given camera ray, $r$, and $\widehat{C_r}$ is the computed color of that camera ray. The cost function considers all camera rays, i.e. the sum is across all rays. Thus, ideally the method utilizes all pixels on the sensors to resolve the aforementioned ambiguity. In some embodiments, the method comprises the step of providing an initial value for the colors and/or likelihoods. As an example, the initial value of a given potential point may be defined as an average color of the image features associated with said potential point. In some embodiments, the potential points form part of a plurality of different mesh surfaces, which represent the different possible depths of each projector ray; thus, they represent different solutions to the correspondence problem. The method may comprise the step of determining a distribution of the color contribution to the image pixels; e.g. given three different surfaces, which one contributes the most to the color of a given pixel. This information may be used to aid in determining the likelihood of a given potential point of a surface being part of the true solution. Thus, the method may comprise the step of determining points in three-dimensional (3D) space that form a solution to a correspondence problem associated with the set of images, wherein the points are determined by comparing pixel colors with computed colors associated with camera rays corresponding to each pixel.

Accordingly, the disclosed method may comprise the step of solving an optimization problem wherein the cost function is minimized in order to determine the numeric value of the color and likelihood of the potential points. The optimization problem may be solved using gradient-based methods. Subsequently, the true solution to the correspondence problem may be found among the potential points based on their likelihood. Specifically, potential points having a high numeric value of the likelihood are considered likely to be part of the solution. Thus, the method may comprise the step of selecting 3D points among the potential points, wherein said selection is based on the likelihood of the potential points, e.g. based on likelihoods exceeding a predetermined threshold value. Alternatively, for each projector ray, the potential point having the highest likelihood may be selected as a 3D point forming part of the true solution. Finally, the method may comprise the step of generating a three-dimensional (3D) representation based on the determined / selected 3D points. The collection of 3D points determined to form part of the true solution may simply collectively form the three-dimensional (3D) representation, e.g. in the form of a point cloud.

The three-dimensional (3D) representation may be a point cloud comprising a plurality of points in 3D space. In some embodiments, the 3D representation is a 3D polygon mesh. The 3D representation based on a single set of images may also be referred to herein as a sub-scan. The 3D representation may only represent a part of the surface of the scanned object. During a scanning session, e.g. using an intraoral scanning device, the scanning device may be moved across the object, typically with a variety of positions and angles, whereby a larger part of the object may be scanned. Thus, during a scanning session the scanning device may be configured to continuously acquire sets of images and generate sub-scans based on said sets of images. The sub-scans may then subsequently be stitched together to form a larger digital 3D model of the scanned object. The disclosed method herein relates in particular to the generation of a sub-scan based on a set of images, wherein the sub-scan constitutes a 3D representation of at least a part of the scanned object.

## Detailed description of the drawings

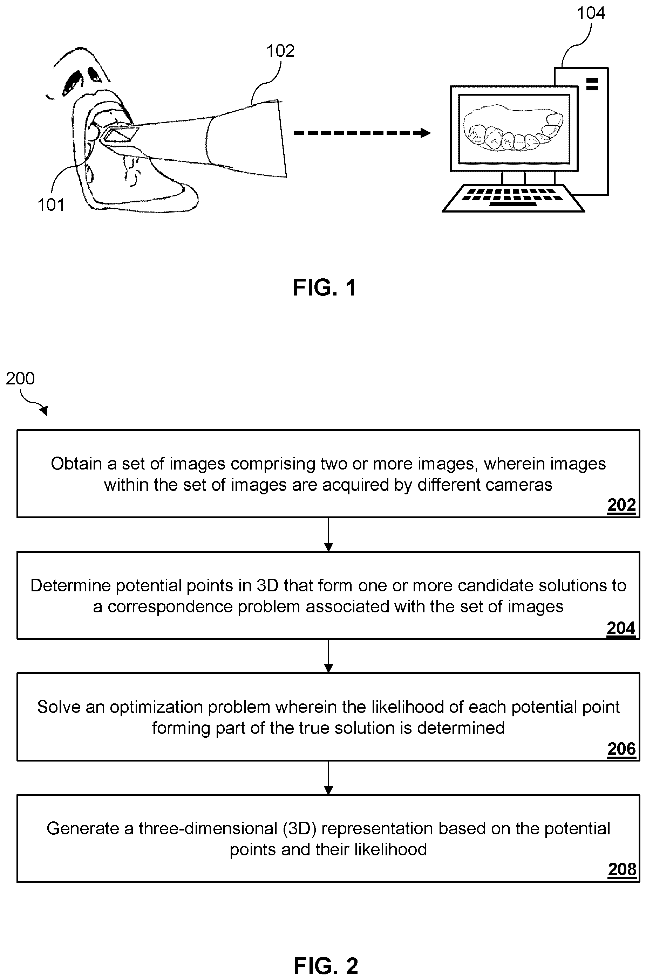

Fig. 1 shows a scanning system according to the present disclosure. The scanning system is configured for generating a three-dimensional (3D) representation of an object 101, such as a dental object. As an example, the object 101 may be at least a part of the oral cavity including any of dentition, gingiva, retromolar trigone, hard palate, soft palate, and floor of the mouth, etc. The scanning system comprises an intraoral scanning device 102 for acquiring a set of images of the scanned object, e.g. within the oral cavity of a person. The scanning system further comprises one or more processors for generating a three-dimensional (3D) representation of the scanned object based on the acquired images. In general, the 3D representation may only represent a part of the object surface, e.g. captured by the field of view of the scanning device 102. Such a 3D representation may also be referred to herein as a sub-scan. The processor(s) may be part of the scanning device 102, or they may be external to the scanning device, or a combination of the two, i.e. such that some processing is performed on the scanning device, and further processing is performed on an external computing device 104. The scanning device may be configured to continuously acquire sets of images and generate sub-scans based on said images. It may further be configured to continuously transmit, either wired or wirelessly, said sub-scans to a computing device 104. The sub-scans may be registered and stitched to each other to form a larger 3D model of the scanned object. Said 3D model may be displayed on a display, e.g. connected to the computing device.

Fig. 2 shows an embodiment of the computer-implemented method disclosed herein. In step 202, a set of images comprising two or more images, such as four images, is obtained, wherein images within the set of images are acquired by different camera units. The set of images may be acquired by the scanning device 102; thus the scanning device may comprise two or more camera units, each camera unit comprising an image sensor. In step 204, potential points in 3D are determined, said potential points forming one or more candidate solutions to the correspondence problem associated with the set of images. In step 206, an optimization problem is solved, wherein the likelihood of each potential point is determined, said likelihood expressing how probable/likely it is that a given potential point forms part of the true solution to the correspondence problem. In step 208, a three-dimensional (3D) representation is generated based on the potential points and their likelihood. The method may comprise further details and steps as disclosed herein.

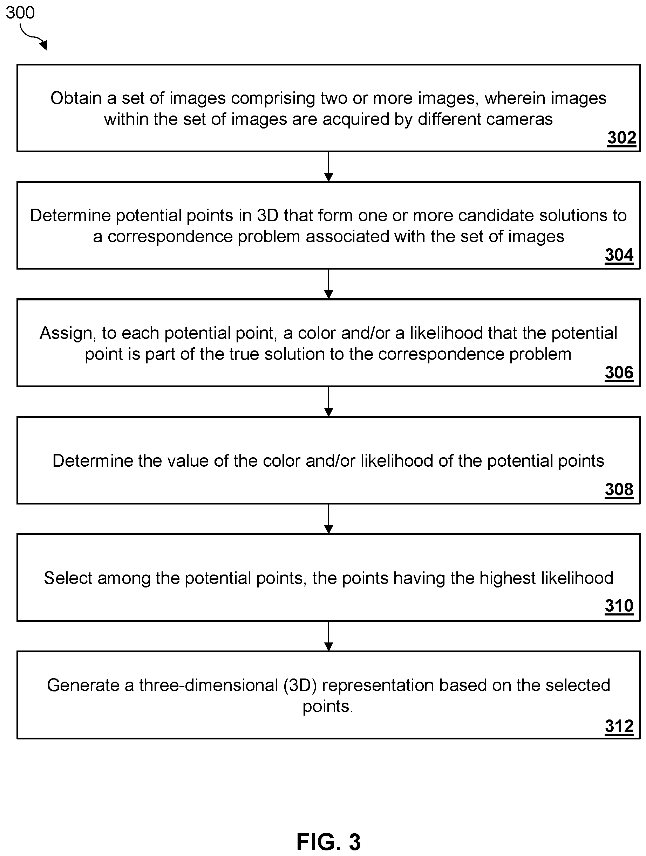

Fig. 3 shows an embodiment of the computer-implemented method disclosed herein. In step 302, a set of images comprising two or more images, such as four images, is obtained, wherein images within the set of images are acquired by different camera units. The set of images may be acquired by the scanning device 102; thus the scanning device may comprise two or more camera units, each camera unit comprising an image sensor. In step 304, potential points in 3D are determined, said potential points forming one or more candidate solutions to the correspondence problem associated with the set of images. In step 306, each potential point is assigned a color and/or a likelihood that the potential point is part of the true solution to the correspondence problem. The color and/or likelihood are parameters / variables; they may be given an initial value for solving the optimization problem, but their final value is determined in step 308. In step 310, the points having the highest likelihood are selected among the potential points. In step 312, a three-dimensional (3D) representation is generated based on the selected points.

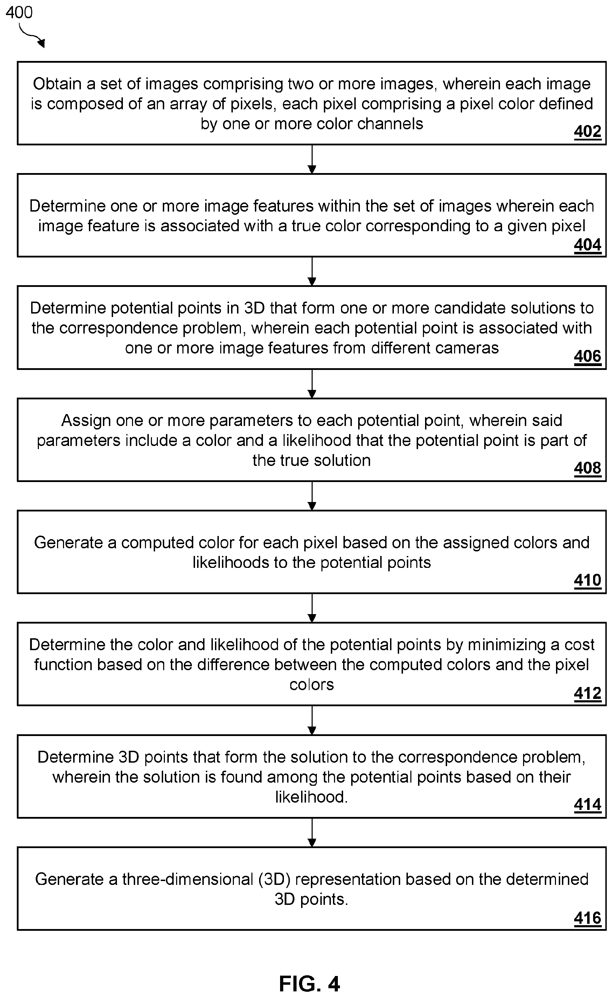

Fig. 4 shows an embodiment of the computer-implemented method disclosed herein. In step 402, a set of images is obtained, the set of images comprising two or more images, wherein each image is composed of an array of pixels, each pixel comprising a pixel color defined by one or more color channels. In step 404, one or more image features within the set of images are determined, wherein each image feature is associated with a true color corresponding to a given pixel. In step 406, potential points in 3D are determined, said potential points forming one or more candidate solutions to the correspondence problem, wherein each potential point is associated with one or more image features from different camera units. In step 408, one or more parameters are assigned to each potential point, wherein said parameters include a color and a likelihood that the potential point is part of the true solution. In step 410, a computed color for each pixel is generated based on the assigned colors and likelihoods to the potential points. In step 412, the values of the color and likelihood of the potential points are determined by minimizing a cost function based on the difference between the computed colors and the pixel colors. The cost function may be minimized using gradient-based methods. In step 414, 3D points that form the solution to the correspondence problem are determined, wherein the points/solution is found among the potential points based on their likelihood. In step 416, a three-dimensional (3D) representation is generated based on the determined 3D points.

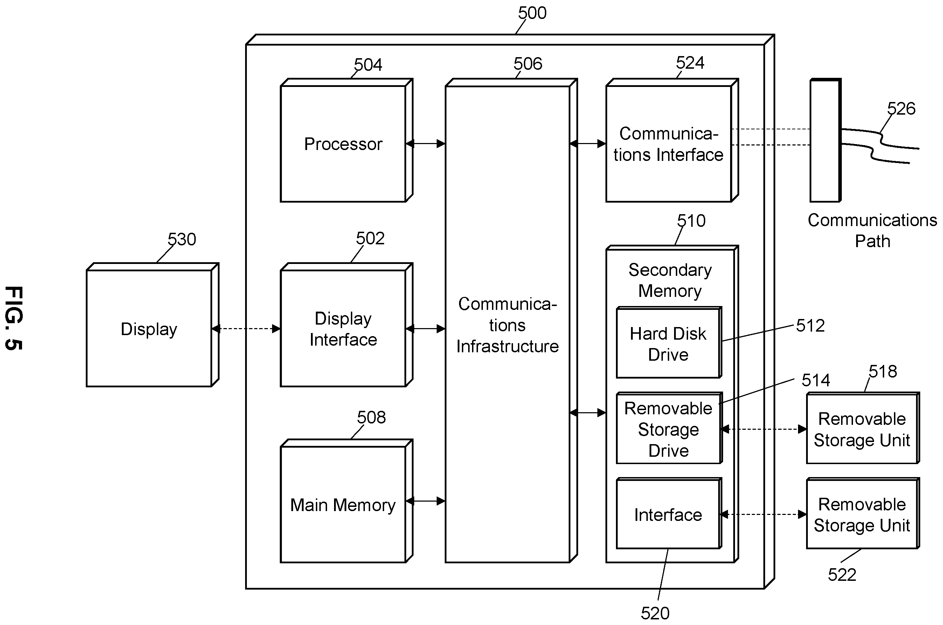

Fig. 5 shows a computer system 500 in which embodiments of the present disclosure, or portions thereof, may be implemented as computer-readable code. For example, the imaging device 102, the computing device 104, and/or a display device may be implemented in the computer system 500 using hardware, software, firmware, non-transitory computer readable media having instructions stored thereon, or a combination thereof and may be implemented in one or more computer systems or other processing systems. Hardware, software, or any combination thereof may embody modules and components used to implement the methods of figs. 2-4.

If programmable logic is used, such logic may execute on a commercially available processing platform configured by executable software code to become a specific purpose computer or a special purpose device (e.g., programmable logic array, application-specific integrated circuit, etc.). A person having ordinary skill in the art may appreciate that embodiments of the disclosed subject matter can be practiced with various computer system configurations, including multi-core multiprocessor systems, minicomputers, mainframe computers, computers linked or clustered with distributed functions, as well as pervasive or miniature computers that may be embedded into virtually any device. For instance, at least one processor device and a memory may be used to implement the above described embodiments.

A processor unit or device as discussed herein may be a single processor, a plurality of processors, or combinations thereof. Processor devices may have one or more processor "cores." The terms "computer program medium," "non-transitory computer readable medium," and "computer usable medium" as discussed herein are used to generally refer to tangible media such as a removable storage unit 518, a removable storage unit 522, and a hard disk installed in hard disk drive 512.

Various embodiments of the present disclosure are described in terms of this example computer system 500. After reading this description, it will become apparent to a person skilled in the relevant art how to implement the present disclosure using other computer systems and/or computer architectures. Although operations may be described as a sequential process, some of the operations may in fact be performed in parallel, concurrently, and/or in a distributed environment, and with program code stored locally or remotely for access by single or multiprocessor machines.

Processor device 504 may be a special purpose or a general purpose processor device specifically configured to perform the functions discussed herein. The processor device 504 may be connected to a communications infrastructure 506, such as a bus, message queue, network, multi-core message-passing scheme, etc. The network may be any network suitable for performing the functions as disclosed herein and may include a local area network (LAN), a wide area network (WAN), a wireless network (e.g., WiFi), a mobile communication network, a satellite network, the Internet, fiber optic, coaxial cable, infrared, radio frequency (RF), or any combination thereof. Other suitable network types and configurations will be apparent to persons having skill in the relevant art. The computer system 500 may also include a main memory 508 (e.g., random access memory, read-only memory, etc.), and may also include a secondary memory 410. The secondary memory 510 may include the hard disk drive 512 and a removable storage drive 514, such as a floppy disk drive, a magnetic tape drive, an optical disk drive, a flash memory, etc.

The removable storage drive 514 may read from and/or write to the removable storage unit 518 in a well-known manner. The removable storage unit 518 may include a removable storage media that may be read by and written to by the removable storage drive 514. For example, if the removable storage drive 514 is a floppy disk drive or universal serial bus port, the removable storage unit 518 may be a floppy disk or portable flash drive, respectively. In one embodiment, the removable storage unit 518 may be non-transitory computer readable recording media.

In some embodiments, the secondary memory 510 may include alternative means for allowing computer programs or other instructions to be loaded into the computer system 500, for example, the removable storage unit 522 and an interface 520. Examples of such means may include a program cartridge and cartridge interface (e.g., as found in video game systems), a removable memory chip (e.g., EEPROM, PROM, etc.) and associated socket, and other removable storage units 522 and interfaces 520 as will be apparent to persons having skill in the relevant art.

Data stored in the computer system 500 (e.g., in the main memory 508 and/or the secondary memory 510) may be stored on any type of suitable computer readable media, such as optical storage (e.g., a compact disc, digital versatile disc, Blu-ray disc, etc.) or magnetic tape storage (e.g., a hard disk drive). The data may be configured in any type of suitable database configuration, such as a relational database, a structured query language (SQL) database, a distributed database, an object database, etc. Suitable configurations and storage types will be apparent to persons having skill in the relevant art.

The computer system 500 may also include a communications interface 524. The communications interface 524 may be configured to allow software and data to be transferred between the computer system 500 and external devices. Exemplary communications interfaces 524 may include a modem, a network interface (e.g., an Ethernet card), a communications port, a PCMCIA slot and card, etc. Software and data transferred via the communications interface 524 may be in the form of signals, which may be electronic, electromagnetic, optical, or other signals as will be apparent to persons having skill in the relevant art. The signals may travel via a communications path 526, which may be configured to carry the signals and may be implemented using wire, cable, fiber optics, a phone line, a cellular phone link, a radio frequency link, etc.

The computer system 500 may further include a display interface 502. The display interface 502 may be configured to allow data to be transferred between the computer system 500 and external display 530. Exemplary display interfaces 502 may include high-definition multimedia interface (HDMI), digital visual interface (DVI), video graphics array (VGA), etc. The display 530 may be any suitable type of display for displaying data transmitted via the display interface 502 of the computer system 500, for example a cathode ray tube (CRT) display, liquid crystal display (LCD), light-emitting diode (LED) display, capacitive touch display, thin-film transistor (TFT) display, etc.

Computer program medium and computer usable medium may refer to memories, such as the main memory 508 and secondary memory 510, which may be memory semiconductors (e.g., DRAMs, etc.). These computer program products may be means for providing software to the computer system 500. Computer programs (e.g., computer control logic) may be stored in the main memory 508 and/or the secondary memory 510. Computer programs may also be received via the communications interface 524. Such computer programs, when executed, may enable computer system 500 to implement the present methods as discussed herein. In particular, the computer programs, when executed, may enable processor device 504 to implement the processes and methods illustrated by figs. 2, 3, and 4 as discussed herein. Accordingly, such computer programs may represent controllers of the computer system 500. Where the present disclosure is implemented using software, the software may be stored in a computer program product and loaded into the computer system 500 using the removable storage drive 514, interface 520, and hard disk drive 512, or communications interface 524.

The processor device 504 may comprise one or more modules or engines configured to perform the functions of the computer system 500. Each of the modules or engines may be implemented using hardware and, in some instances, may also utilize software, such as corresponding to program code and/or programs stored in the main memory 508 or secondary memory 510. In such instances, program code may be compiled by the processor device 504 (e.g., by a compiling module or engine) prior to execution by the hardware of the computer system 500. For example, the program code may be source code written in a programming language that is translated into a lower level language, such as assembly language or machine code, for execution by the processor device 504 and/or any additional hardware components of the computer system 500. The process of compiling may include the use of lexical analysis, preprocessing, parsing, semantic analysis, syntax-directed translation, code generation, code optimization, and any other techniques that may be suitable for translation of program code into a lower level language suitable for controlling the computer system 500 to perform the functions disclosed herein. It will be apparent to persons having skill in the relevant art that such processes result in the computer system 500 being a specially configured computer system 500 uniquely programmed to perform the functions discussed above.

Fig. 6 shows a visualization of candidate solutions to the correspondence problem associated with a set of images. The candidate solutions are formed by connecting potential points in a mesh. Subsequently, the mesh has been colored according to the likelihood of the potential points, such that the most likely surface of the scanned object is visualized among all the possible surfaces. In this case, the most likely surface, considered to be the true solution to the correspondence problem, is colored in orange.

**Claims**

1. A computer-implemented method for generating a three-dimensional (3D) representation of a surface of a dental object (101), the method comprising the steps of:

   - obtaining a set of images comprising two or more images acquired by a triangulation-based intraoral scanning device (102) comprising multiple camera units and one or more projector units configured to project a pattern on a surface of the dental object, wherein images within the set of images are acquired by different camera units, wherein each image comprises a plurality of image features, wherein each image is composed of an array of pixels, wherein each pixel comprises a pixel color defined by one or more color channels;
   - determining points in three-dimensional (3D) space that form a solution to a correspondence problem associated with the set of images; and
   - generating the three-dimensional (3D) representation based on the determined 3D points,
   WHEREIN the points are determined by
   - determining potential points in 3D space that form one or more candidate solutions to the correspondence problem based on triangulation;
   - assigning one or more parameters to each potential point, wherein said parameters include a color and a likelihood that the potential point is part of the solution to the correspondence problem;
   - generating a computed color for each camera ray corresponding to a pixel, wherein the computed color is based on the assigned colors and likelihoods; and
   - comparing pixel colors with the computed colors associated with camera rays corresponding to each pixel by determining a numeric value of the color and likelihood of the potential points by minimizing a cost function based on the difference between the computed colors and the pixel colors, wherein the solution to the correspondence problem is found among the potential points based on their numeric value of their likelihood.

2. The computer-implemented method according to claim 1, wherein images within a set of images are acquired simultaneously.

3. The computer-implemented method according to claims 1 or 2, wherein the method further comprises the step of determining one or more image features within the set of images, wherein each potential point is determined by triangulation based on the determined image features.

4. A scanning system comprising:

   - a triangulation-based intraoral scanning device (102) comprising one or more projector units configured to project a pattern on a surface of the dental object (101) and two or more camera units configured to acquire the set of images, wherein the set of images comprises at least one image from each camera unit;
   - one or more processors (504) configured to perform the steps of the method according to any of the claims 1-3.

5. The scanning system according to claim 4, wherein the projected pattern is static.

6. The scanning system according to any of claims 4-5, wherein the projected pattern is a polygonal pattern comprising at least 3000 pattern features.

**Patentansprüche**

1. Computerimplementiertes Verfahren zum Erzeugen einer dreidimensionalen (3D) Darstellung einer Oberfläche eines dentalen Objekts (101), wobei das Verfahren die Schritte umfasst

   - Erhalten eines Satzes von Bildern, der zwei oder mehr Bilder umfasst, die von einer triangulationsbasierten intraoralen Scanvorrichtung (102) aufgenommen wurden, die mehrere Kameraeinheiten und eine oder mehrere Projektoreinheiten umfasst, die dazu konfiguriert sind, ein Muster auf eine Oberfläche des dentalen Objekts zu projizieren, wobei Bilder innerhalb des Bildsatzes von unterschiedlichen Kameraeinheiten aufgenommen werden, wobei jedes Bild eine Mehrzahl von Bildmerkmalen umfasst, wobei jedes Bild aus einem Array von Pixeln zusammengesetzt ist, wobei jedes Pixel eine Pixelfarbe umfasst, die durch einen oder mehrere Farbkanäle definiert ist;
- Bestimmen von Punkten im dreidimensionalen (3D) Raum, die eine Lösung für ein dem Bildsatz zugeordnetes Korrespondenzproblem bilden; und
- Erzeugen der dreidimensionalen (3D) Darstellung auf der Grundlage der bestimmten 3D-Punkte, WOBEI die Punkte bestimmt werden durch
- Bestimmen potenzieller Punkte im 3D-Raum, die auf Basis von Triangulation eine oder mehrere Kandidatenlösungen für das Korrespondenzproblem bilden;
- Zuordnen eines oder mehrerer Parameter zu jedem potenziellen Punkt, wobei diese Parameter eine Farbe und eine Wahrscheinlichkeit umfassen, dass der potenzielle Punkt Teil der Lösung des Korrespondenzproblems ist;
- Erzeugen einer berechneten Farbe für jeden Kamerastrahl, der einem Pixel entspricht, wobei die berechnete Farbe auf den zugeordneten Farben und Wahrscheinlichkeiten basiert; und
- Vergleichen von Pixelfarben mit den berechneten Farben, die den Kamerastrahlen zugeordnet sind, die jedem Pixel entsprechen, durch Bestimmen eines numerischen Werts der Farbe und der Wahrscheinlichkeit der potenziellen Punkte durch Minimieren einer Kostenfunktion auf Basis der Differenz zwischen den berechneten Farben und den Pixelfarben, wobei die Lösung des Korrespondenzproblems unter den potenziellen Punkten auf Grundlage deren numerischen Werts derer Wahrscheinlichkeit gefunden wird.

2. Computerimplementiertes Verfahren nach Anspruch 1, wobei Bilder innerhalb eines Bildsatzes gleichzeitig aufgenommen werden.

3. Computerimplementiertes Verfahren nach Anspruch 1 oder 2, wobei das Verfahren ferner den Schritt des Bestimmens eines oder mehrerer Bildmerkmale innerhalb des Bildsatzes umfasst, wobei jeder potenzielle Punkt durch Triangulation auf Grundlage der bestimmten Bildmerkmale bestimmt wird.

4. Scansystem, umfassend:

- eine triangulationsbasierte intraorale Scanvorrichtung (**102**), die eine oder mehrere Projektoreinheiten umfasst, die dazu konfiguriert sind, ein Muster auf eine Oberfläche des dentalen Objekts (**101**) zu projizieren, und zwei oder mehr Kameraeinheiten, die dazu konfiguriert sind, den Bildsatz aufzunehmen, wobei der Bildsatz mindestens ein Bild von jeder Kameraeinheit umfasst;
- einen oder mehrere Prozessoren (**504**), die dazu konfiguriert sind, die Schritte des Verfahrens nach einem der Ansprüche 1 bis 3 auszuführen.

5. Scansystem nach Anspruch 4, wobei das projizierte Muster statisch ist.

6. Scansystem nach einem der Ansprüche 4 bis 5, wobei das projizierte Muster ein polygonales Muster ist, das mindestens 3000 Mustermerkmale umfasst.

**Revendications**

1. Procédé mis en œuvre par ordinateur pour générer une représentation tridimensionnelle (3D) d'une surface d'un objet dentaire (**101**), le procédé comprenant les étapes consistant à :

- l'obtention d'un ensemble d'images comprenant deux ou plusieurs images acquises par un dispositif de balayage intra-oral à base de triangulation (**102**) comprenant plusieurs unités de caméra et une ou plusieurs unités de projecteur configurées pour projeter un motif sur une surface de l'objet dentaire, dans lequel des images au sein de l'ensemble d'images sont acquises par différentes unités de caméra, dans lequel chaque image comprend une pluralité de caractéristiques d'image, dans lequel chaque image est composée d'un réseau de pixels, dans lequel chaque pixel comprend une couleur de pixel définie par un ou plusieurs canaux de couleur ;
- la détermination de points dans l'espace tridimensionnel (3D) qui forment une solution à un problème de correspondance associé à l'ensemble d'images ; et
- la génération de la représentation tridimensionnelle (3D) à partir des points 3D déterminés, dans lequel les points sont déterminés par
- la détermination de points potentiels dans l'espace 3D qui forment une ou plusieurs solutions candidates au problème de correspondance sur la base d'une triangulation ;
- l'assignation d'un ou plusieurs paramètres à chaque point potentiel, dans lequel lesdits paramètres incluent une couleur et une probabilité que le point potentiel fasse partie de la solution au problème de correspondance ;

- la génération d'une couleur calculée pour chaque rayon de caméra correspondant à un pixel, dans lequel la couleur calculée est basée sur les couleurs et les probabilités assignées ; et

- la comparaison de couleurs de pixels avec les couleurs calculées associées aux rayons de caméra correspondant à chaque pixel en déterminant une valeur numérique de la couleur et de la probabilité des points potentiels par la minimisation d'une fonction de coût basée sur la différence entre les couleurs calculées et les couleurs de pixels, dans lequel la solution au problème de correspondance est trouvée parmi les points potentiels sur la base de la valeur numérique de leur probabilité.

2. Procédé mis en œuvre par ordinateur selon la revendication 1, dans lequel des images au sein d'un ensemble d'images sont acquises simultanément.

3. Procédé mis en œuvre par ordinateur selon les revendications 1 ou 2, dans lequel le procédé comprend en outre l'étape consistant à déterminer une ou plusieurs caractéristiques d'image au sein de l'ensemble d'images, dans lequel chaque point potentiel est déterminé par triangulation à partir des caractéristiques d'image déterminées.

4. Système de balayage comprenant :

- un dispositif de balayage intra-oral à base de triangulation (102) comprenant une ou plusieurs unités de projecteur configurées pour projeter un motif sur une surface de l'objet dentaire (101) et deux ou plusieurs unités de caméra configurées pour acquérir l'ensemble d'images, dans lequel l'ensemble d'images comprend au moins une image provenant de chaque unité de caméra ;

- un ou plusieurs processeurs (504) configurés pour réaliser les étapes du procédé selon l'une quelconque des revendications 1 à 3.

5. Système de balayage selon la revendication 4, dans lequel le motif projeté est statique.

6. Système de balayage selon l'une quelconque des revendications 4 à 5, dans lequel le motif projeté est un motif polygonal comprenant au moins 3000 caractéristiques de motif.

**FIG. 1**

200

Obtain a set of images comprising two or more images, wherein images within the set of images are acquired by different cameras

**202**

Determine potential points in 3D that form one or more candidate solutions to a correspondence problem associated with the set of images

**204**

Solve an optimization problem wherein the likelihood of each potential point forming part of the true solution is determined

**206**

Generate a three-dimensional (3D) representation based on the potential points and their likelihood

**208**

**FIG. 2**

300

Obtain a set of images comprising two or more images, wherein images within the set of images are acquired by different cameras

302

Determine potential points in 3D that form one or more candidate solutions to a correspondence problem associated with the set of images

304

Assign, to each potential point, a color and/or a likelihood that the potential point is part of the true solution to the correspondence problem

306

Determine the value of the color and/or likelihood of the potential points

308

Select among the potential points, the points having the highest likelihood

310

Generate a three-dimensional (3D) representation based on the selected points.

312

**FIG. 3**

400

Obtain a set of images comprising two or more images, wherein each image is composed of an array of pixels, each pixel comprising a pixel color defined by one or more color channels
**402**

Determine one or more image features within the set of images wherein each image feature is associated with a true color corresponding to a given pixel
**404**

Determine potential points in 3D that form one or more candidate solutions to the correspondence problem, wherein each potential point is associated with one or more image features from different cameras
**406**

Assign one or more parameters to each potential point, wherein said parameters include a color and a likelihood that the potential point is part of the true solution
**408**

Generate a computed color for each pixel based on the assigned colors and likelihoods to the potential points
**410**

Determine the color and likelihood of the potential points by minimizing a cost function based on the difference between the computed colors and the pixel colors
**412**

Determine 3D points that form the solution to the correspondence problem, wherein the solution is found among the potential points based on their likelihood.
**414**

Generate a three-dimensional (3D) representation based on the determined 3D points.
**416**

**FIG. 4**

FIG. 5

FIG. 6

REFERENCES CITED IN THE DESCRIPTION

**Patent documents cited in the description**

- US 20200404243 A1
- US 20220280269 A1

- WO 2017003673 A1